# EUROPEAN PATENT APPLICATION

(11) **EP 4 205 598 A1**
(43) Date of publication of application: **05.07.2023**
(21) Application number: 21860221.7
(22) Date of filing: 18.08.2021
(51) Int. Cl.: A45D 44/00

(54) **SELF-DRIVING PULSE MICROCURRENT COSMETIC MASK**

(30) Priority: 28.08.2020 CN 202010881998; 15.06.2021 CN 202121325961 U
(71) Applicant: Jiaxing Juechuang Technology Co., Ltd., Jiaxing, Zhejiang 314031 (CN); Yangtze River Delta (Jiaxing) Nano Applied Technology Research Institute, Jiaxing, Zhejiang 314031 (CN)
(72) Inventor: CAO, Xia, Beijing 101407 (CN); WANG, Jue, Beijing 101407 (CN)
(74) Representative: dompatent von Kreisler Selting Werner - Partnerschaft von Patent- und Rechtsanwälten mbB
(86) International application number: PCT/CN2021/113138
(87) International publication number: WO 2022/042383

(57) **Abstract**

The present invention provides a self-driving pulse microcurrent cosmetic mask, relating to the technical field of cosmetic treatment. The self-driving pulse microcurrent cosmetic mask comprises two parts: a mask body and a cosmetic brush. Through the constant interaction between the mask body and the cosmetic brush, Maxwell's displacement electric field and pulse microcurrent are generated to stimulate the expansion of blood vessels on the skin, improve the circulation of blood and lymphatic fluid, accelerate cell metabolism and enhance skin vitality; open the gap between skin cells and promote the absorption of nutrients; clean the skin and kill bacteria and mites on the skin, etc., thus achieving effects of anti-aging, skin tightening and skin whitening and delicate. The present invention does not need any power source, is easy to carry and operate, and is thus suitable for people of all ages. It is a brand new technology, the first high-tech product in the world, which applies high technology to daily skin care, bringing new changes to our life and revitalizing our skin.

## Description

### TECHNICAL FIELD

The present invention relates to the field of cosmetic technologies, in particular to a self-driving pulse microcurrent cosmetic mask.

### BACKGROUND

With the development of technology, cosmetic technology is also constantly innovating, and research has found that the use of microcurrent can get anti-aging, skin tightening and other effects, the microcurrent cosmetic instrument, microcurrent mask and other products based on this principle are popular. However, most microcurrent cosmetic devices on the market require periodic charging, which greatly limits their convenience, and such products currently on the market are expensive and unsuitable for popularisation among the general public.

The present invention provides a self-driving pulse microcurrent cosmetic mask which is simple to operate, easy to carry, does not require any power source, and does not have the hidden danger of excessive current. It can be disassembled and washed for repeated use, and is suitable for people of all ages. This is a brand new technology application, applying our new cutting-edge technology to the field of beauty and skincare, so that we can protect our skin anytime and anywhere to facilitate our life and make us more beautiful and healthy.

### SUMMARY

The technical problem to be solved by the present invention is to provide a self-driving pulse microcurrent cosmetic mask. It includes a mask body and a cosmetic brush. The mask body and the cosmetic brush are respectively independent devices with complete structure. The cosmetic brush and the mask body are in constant force, generating Maxwell's displacement electric field and Maxwell's displacement current, which achieve stimulation of the skin from point to plane, avoiding local skin damage and allergy, and making the skin delicate and revitalized.

The cosmetic mask in this new technology can generate a short-circuit current of up to 505050 microamps and an open-circuit voltage of up to 1000-1200 volts. Using the cosmetic mask made from special materials in this technology, the mask body generates a pulse electric field and a pulse current that can light up a 1-watt light bulb. The microcurrent cosmetic mask provided by the present invention works on the joint action of Maxwell's displacement electric field and the point current, which has a more uniform stimulation of the skin than the action of the point current alone on the market, avoiding local skin allergy and damage.

The cosmetic mask can be used anytime, anywhere, with or without make-up, on dry or wet surfaces. It is green, safe and environmentally friendly.

The self-driving pulse microcurrent cosmetic mask can not have a cosmetic brush, so the hand interacts directly with the mask body to generate pulse microcurrent, stimulating the skin's blood circulation and rejuvenating the skin.

The self-driving pulse microcurrent cosmetic mask does not need to be connected to an external power source, there is no safety risk of leakage and there is little irritation and damage to the skin.

The self-driving pulse microcurrent cosmetic masks can be applied to all parts of the body, mainly including: face, chest, hands, legs, neck, feet, etc., especially to stimulate mammary development, tighten and lift the skin, reduce neck lines and fine lines.

The mask body is manufactured from flexible or rigid materials, mainly including textile materials, silk materials, cotton materials, chemical fiber materials, polymer materials, organic materials, inorganic materials, organic-inorganic composites. The size and shape of mask body can be adjusted and changed according to the area of use.

The mask body can be a single-layer membrane or a multi-layer membrane, and the mask body can be a mesh structure, a dense structure, or a porous structure.

The structure of the surface of the cosmetic brush in contact with the mask body can be made bristle-like, a flat membrane, or a porous structure.

The inner surface of the mask body is in contact with the skin. Through the interaction between the mask body and the cosmetic brush or the hand, the pulse microcurrent is generated by the mask body to stimulate the blood circulation in the skin.

The effect of the self-driving pulse microcurrent cosmetic mask is related to the contact force, contact time and contact area of the mask body and the cosmetic brush or hand. The contact force and time are determined according to the individual skin's ability to withstand.

In addition to improving the condition of the skin, the self-driving pulse microcurrent cosmetic mask can also kill and remove bacteria, mites and other microorganisms from the skin, leaving the skin clean, fresh and revitalized.

The color of the self-driving pulse microcurrent cosmetic mask can be changed to suit the client's requirements.

The mask is adapted to other parts of the face by changing its shape, keeping other structures intact, to make one or more of the cosmetic patches for the nasolabial folds, forehead, nose, neck, lips and plump cheeks

### BRIEF DESCRIPTION OF DRAWINGS

FIG. 1 is a schematic diagram of a self-driving pulse microcurrent cosmetic mask according to the present invention;
FIG. 2 is a principle diagram of a self-driving pulse microcurrent cosmetic mask according to the present invention;
FIG. 3 is a schematic diagram of a self-driving pulse microcurrent cosmetic mask according to embodiment 1 of the present invention;
FIG. 4 is a schematic diagram of a self-driving pulse microcurrent cosmetic mask according to embodiment 2 of the present invention;
FIG. 5 is a schematic diagram of a self-driving pulse microcurrent cosmetic mask according to embodiment 3 of the present invention;
FIG. 6 is a schematic diagram of the open circuit voltage generated by a self-driving pulse microcurrent cosmetic mask;
FIG. 7 is a schematic diagram of the short-circuit current generated by a self-driving pulse microcurrent cosmetic mask;
FIG. 8 is a schematic diagram of a light bulb powered by a self-driving pulse microcurrent cosmetic mask.

In the drawings:
1 Base;
2 Contact surface;
3 Mask body;
4 Cosmetic brush;
5 Mask type;
6 Hand film or glove type;
7 Leg film type;
8 Human leg.

### DESCRIPTION OF EMBODIMENTS

For a better clarity of the technical problem to be solved by the present invention as well as the technical solutions and advantages of the present invention, a detailed description will be given below with reference to the accompanying drawings and specific embodiments.

The present invention provides a self-driving pulse microcurrent cosmetic mask.

The self-driving pulse microcurrent cosmetic mask includes a mask body and a cosmetic brush. The mask body and the cosmetic brush are respectively independent devices with complete structure. The cosmetic brush and the mask body are in contact with each other to generate pulse microcurrent, which stimulates the blood circulation of the skin capillaries, shrink the pores and makes the skin tight and delicate. The cosmetic mask can be designed without the cosmetic brush. Maxwell displacement current can be generated directly through the contact or friction between other objects and the mask body to achieve the effect of beauty.

The cosmetic mask can be used anytime, anywhere, with or without make-up, on dry or wet surfaces. It is green, safe and environmentally friendly.

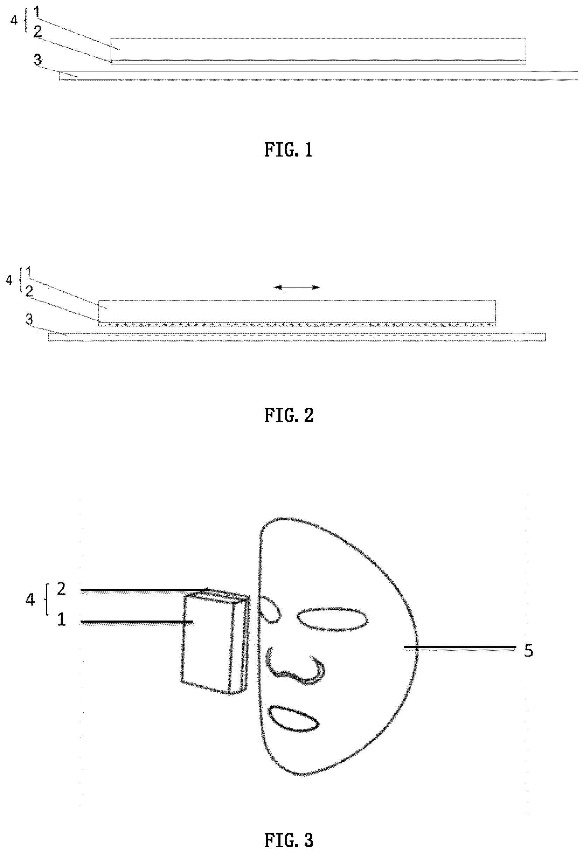

As shown in FIG. 1 and FIG. 2, the schematic diagram and the principle diagram of the cosmetic mask are shown respectively. The cosmetic mask is different from the microcurrent beauty devices in the current market. The cosmetic mask works on a joint action of Maxwell's displacement electric field and the point current, which has a more uniform and continuous stimulation of the skin than the action of the point current alone, avoiding local skin damage and allergy.

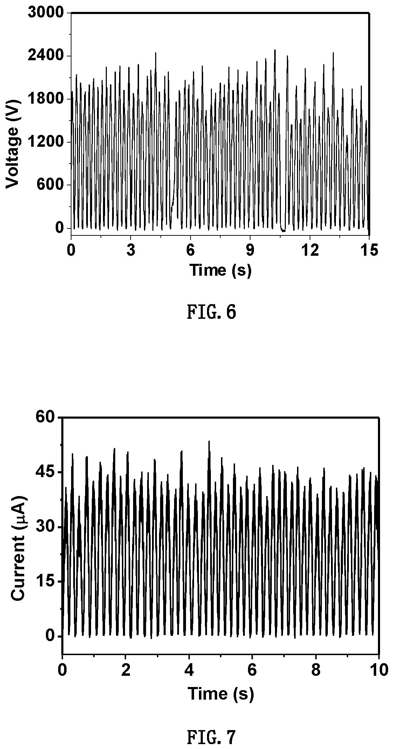

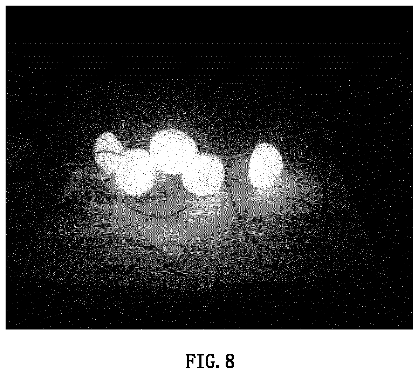

As shown in FIG. 6, FIG. 7 and FIG. 8, the cosmetic mask in this new technology can generate a short-circuit current of up to 505050 microamps and an open-circuit voltage of up to 1000-1200 volts. Using the cosmetic mask made from special materials in this technology, the mask body generates a pulse electric field and a pulse current that can light up a 1-watt light bulb.

The self-driving pulse microcurrent cosmetic masks can be applied to all parts of the body, mainly including: face, chest, hands, legs, neck, feet, etc., especially to stimulate mammary development, tighten and lift the skin, and reduce neck lines and fine lines.

A description is given below in conjunction with the specific embodiments.

### Embodiment 1

As shown in FIG. 3, A self-driving pulse microcurrent cosmetic mask in this embodiment is used for facial skin maintenance. The self-driving pulse microcurrent cosmetic mask is made into a mask type 5, so that it fits the skin of the face. The mask can generate Maxwell displacement electric field and Maxwell displacement current by using a cosmetic brush or other objects to rub or touch on the surface of the mask. It stimulates the blood circulation of the facial skin, shrinks the pores, and makes the skin tight, red and delicate.

### Embodiment 2

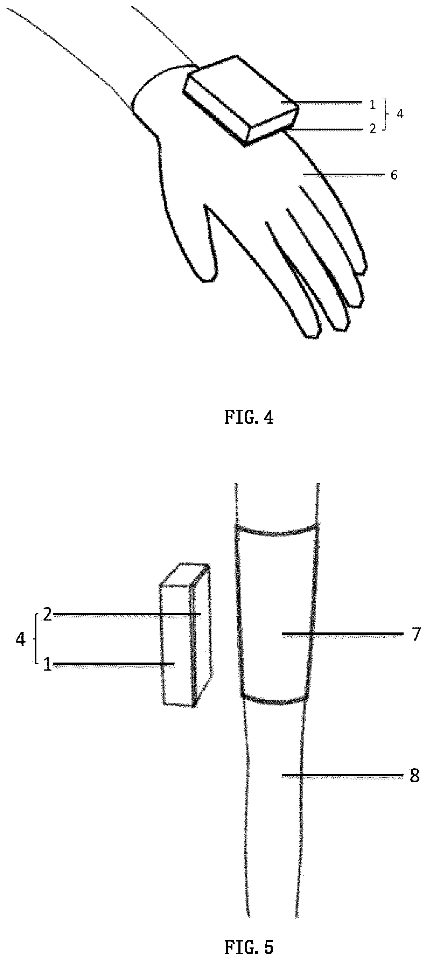

As shown in FIG. 4, a self-driving pulse microcurrent cosmetic mask in this embodiment is used for the maintenance and care of hand skin. The self-driving pulse microcurrent cosmetic mask is made into a hand film or glove type 6, so that it fits the skin of the hand. The hand film or glove generates Maxwell displacement electric field and Maxwell displacement current by using a cosmetic brush or other objects to rub or touch the surface of the hand film or glove. It stimulates the blood circulation of the hand skin, shrinks the pores and makes the hand skin firm and delicate.

### Embodiment 3

As shown in FIG. 5, a self-driving pulse microcurrent cosmetic mask in this embodiment is used for the maintenance and care of the leg skin. The self-driving pulse microcurrent cosmetic mask is made into a leg film type 7, so that it fits the skin of the human leg 8. The leg film generates Maxwell displacement electric field and Maxwell displacement current by using a cosmetic brush or other objects to rub or touch the surface of the leg film. It can stimulate the blood circulation of the leg skin, shrinks the pores, and makes the leg skin tight and delicate.

## Claims

1. A self-driving pulse microcurrent cosmetic mask, comprising two parts: a mask body (3) and a cosmetic brush (4). The mask body and the cosmetic brush are respectively independent devices with complete structure. The cosmetic brush and the mask body are in constant force, generating Maxwell's displacement electric field and Maxwell's displacement current, which achieve stimulation of the skin from point to plane, avoiding local skin damage and allergy, but also making the skin delicate and revitalized.

2. The self-driving pulse microcurrent cosmetic mask according to claim 1, wherein the mask is not equipped with a cosmetic brush, but directly generates pulse microcurrent through the interaction of other objects with the body of the mask, stimulating the blood circulation of the skin and stimulating its vitality.

3. The self-driving pulse microcurrent cosmetic mask according to claim 1, wherein the mask does not require an external power supply and relies on the force generated by the constant friction between the cosmetic brush or other objects and the body of the mask to generate pulsing micro-currents, which are not harmful to the body but have a stimulating effect on the skin.

4. The self-driving pulse microcurrent cosmetic mask according to claim 1, wherein the mask is applied to all parts of the body, including: face, chest, hands, legs, neck and feet, stimulating blood circulation and firming and lifting the skin.

5. The self-driving pulse microcurrent cosmetic mask according to claim 1, wherein the mask body is made of one of the flexible, rigid or rigid-flexible materials and the size and shape of the mask body can be adapted to the area of use.

6. The self-driving pulse microcurrent cosmetic mask according to claim 1, wherein the mask body is no less than one layer.

7. The self-driving pulse microcurrent cosmetic mask according to claim 1, wherein the inner surface of the mask body interacts with the skin under a force including friction, contact, collision or tapping. Through the interaction between the mask body and the cosmetic brush or the hand, the pulse microcurrent is generated by the mask body to stimulate the blood circulation in the skin.

8. The self-driving pulse microcurrent cosmetic mask according to claim 1, wherein the cosmetic brush comprises a substrate (1) and a contact surface (2) with a bristle-like, flat film or porous structure.

9. The self-driving pulse microcurrent cosmetic mask according to claim 1, wherein the mask is adapted to other parts of the face by changing its shape, keeping other structures intact, to make one or more of the cosmetic patches for the nasolabial folds, forehead, nose, neck, lips and plump cheeks.
